# EUROPÄISCHE PATENTSCHRIFT

(11) **EP 0 428 965 B1**
(45) Veröffentlichungstag und Bekanntmachung des Hinweises auf die Patenterteilung: **02.05.1997**
(21) Anmeldenummer: 90121562.4
(22) Anmeldetag: 10.11.1990
(51) Int. Cl.: C08L 3/02, A61K 47/36, A61K 9/20

(54) **Modifizierte Stärke und Verfahren zu ihrer Herstellung**
Modified starch and process to obtain the same
Amidon modifié et procédé de préparation

(30) Priorität: 14.11.1989 CH 4099/89
(43) Veröffentlichungstag der Anmeldung: 29.05.1991
(73) Patentinhaber: Gergely, Gerhard, Dr., A-1050 Wien (AT)
(72) Erfinder: Gergely, Gerhard, Dr., A-1050 Wien (AT); Gergely, Thomas, Dr., A-1050 Wien (AT); Gergely, Irmgard, A-1050 Wien (AT)
(74) Vertreter: Büchel, Kurt F., Dr.

(56) Entgegenhaltungen:
- EP-A- 0 070 970
- EP-A- 0 159 631
- CHEMICAL ABSTRACTS, Band 99, Nr. 16, 17. Oktober 1983, Seite 360, Zusammenfassung Nr. 128367m, Columbus, Ohio, US; & JP-A-58 27 774 (TAKEDA CHEMICAL INDUSTRIES LTD) 11-06-1983

## Beschreibung

In der pharmazeutischen Industrie besteht ein steigender Bedarf an Tabletten-Hilfsstoffen. Insbesondere seit die bisher üblichen Hilfsstoffe wie Lactose (wegen Unverträglichkeitserscheinungen), Mannitol, Xylitol, etc. wegen laxativer Wirkungen, immer mehr abgelehnt werden, ist es notwendig, Tabletten-Hilfsstoffe zu entwickeln, die keinerlei Kritik an ihren pharmakologischen Eigenschaften erlauben.

Ein idealer Tabletten-Hilfsstoff wäre Stärke, die physiologisch unbedenklich und Bestandteil vieler natürlicher Lebensmittel ist.

Die Einsatzmöglichkeiten von Stärke in Tablettenformulierungen sind begrenzt, da sie an sich direkt schlecht verpressbar ist, weil sie aufgrund ihrer Struktur über keinerlei Bindungsfähigkeiten verfügt. Um eine gewünschte Verpressbarkeit zu erzeilen, müsste man Stärke bzw. stärkehaltige Produkte **wie zum Beispiel auf Seite 1578 unter "Binders" in Remington's Pharmaceutical Sciences, Mack Publishing Co. 1975, bzw. in EP-A-0366621 auf Seite 5 beschrieben**, granulieren. Dafür hat man bisher hauptsächlich wässrige Lösungen von Dextrin, Gummi arabicum, Polyvinylpyrrolidon (PVP) und andere Bindemittel verwendet.

**In EP-A-0070970 wird Stärke durch Anschwellen und Vorvernetzung in Wasser, unter Zugabe eines relativ hohen Anteils an chemischem Vernetzungsmittel, vorbehandelt, um bessere Zerfallseigenschaften der Stärke zu erreichen.**

**Im Gegensatz zu US-A-4673438, US-A-4337181 und EP-A-400531, die alle verschiedene Methoden zur formgebenden Verarbeitung von Stärke, z.B. im Spritzguss, beschreiben, wobei die gute Verarbeitbarkeit der zusammengemischten Ausgangsstoffe durch eine Plastifizierung unter Druck und Temperatur erfolgt, wird** **erfindungsgemäss die Verpressbarkeit von Stärke für die Tablettierung verbessert.**

Stärke kann jedoch als Tabletten-Hilfsstoff erfindungsgemäss verwendet werden, wenn man die Stärke mit Substanzen belädt, die in solchen Lösungsmitteln gelöst sind, die Stärke nicht hydrolisieren können.

Beispielsweise sind organische Säuren oder auch Polyvinylpyrrolidone, speziell solche mit höherer Kettenlänge, dazu geeignet, der Stärke Bindungseigenschaften zu verleihen. Bei diesen Bindungseigenschaften bleibt der Sprengcharakter der Stärke grösstenteils erhalten; es liegt lediglich an der Menge der Beladung, wie weit die Stärke noch quellfähig und zerfallfähig ist, oder nur mehr als sonstiger Tabletten-Hilfsstoff, wie z.B. Tabletten-Füllstoff, ohne Zerfall eingesetzt werden kann.

Natürlich kann man solche modifizierten Stärken auch in Kombination mit anderen Zerfallsstoffen, wie beispielsweise kreuzvernetztem Polyvinylpolypyrrolidon verwenden, so dass die Stärke sowohl als Sprengmittel als auch als sonstiges Tabletten-Hilfsmittel, beispielsweise als Füllmittel, eingesetzt werden kann.

Hinsichtlich der Quellfähigkeit von Sprengmitteln gibt der Aufsatz von A.H.Bronnsack in Pharm.Ind.38/12,1181-1185 1976), insbesondere in der Einleitung, einen guten Überblick.

Will man daher - aus welchen Gründen immer - Tabletten mit einem höheren Zusatz an Stärke (gleichgültig welcher Herkunft) herstellen, dann ergeben sich aufgrund der schlechten Presseigenschaften geringere Tablettenhärten.

Dies kann durch Massnahmen vermieden werden, wie sie beispielsweise im Kennzeichen des Anspruches 1 beschrieben sind. Vorteilhafte Weiterbildungen der Erfindung, insbesondere auch ein Verfahren zur Herstellung der erfindungsgemäss modifizierten Stärke, sind in den Kennzeichen der Unteransprüche beschrieben.

Zum Beispiel wird die Stärke mit einer Säure in alkoholischer Lösung behandelt. Versetzt man 100 Teile Reisstärke mit 10 Teilen Weinsäure in 30 Teilen Alkohol, dann wird nach dem Verdampfen des Alkohols die Weinsäure innerhalb der Stärke kristallisieren und der Stärke wesentlich verbesserte Presseigenschaften verleihen. Da durch die Alkoholzugabe die Stärke nicht quillt, sondern beim Trocknen sogar von einem ursprünglichen Wassergehalt von 12 - 15% auf 6-7% zurückgeht, ist dieser Schritt besonders geeignet, auch schwierigere Formulierungen zu harten Tabletten zu verpressen.

Solche Stärkebehandlungen mit verschiedenen Substanzen können in normalen Granulatoren durchgeführt werden; besonders vorteilhaft ist aber das Evakuieren der Stärke bei Temperaturen von 40-50° C, das Einsaugen der alkoholischen Lösung in die Stärke, wodurch die Lösung teilweise sogar in die Zellstruktur der Stärkekörner eindringen kann, und das nachherige Verdampfen des Lösungsmittels im Vakuum. Auf diese Weise gelingt es, verschiedene Substanzen ausgezeichnet innerhalb der Stärkekörner zu verankern.

Verwendet man die Vakuumbeladung von Stärken, dann lassen sich sogar zur Verbesserung der Presseigenschaften alkoholische PVP-Lösungen in der Stärke einbauen, wobei insbesondere naturgemäss längerkettiges PVP(K 90) besonders geeignet ist.

### Beispiel 1:

500 Teile Stärke werden im Mischkessel auf 40° C erwärmt und mit einer Lösung von 100 Teilen Alkohol und 50 Teilen Zitronensäure versetzt. Die Lösung wird hernach im Hordentrockner oder Wirbelschichttrockner auf 6% Feuchtigkeit getrocknet.

Auch hier gilt der Umstand, dass die leicht lösliche Zitronensäure bei Verwendung zur Stärkebeladung zwar härtere Tabletten, aber eine 3-4-fache Verlängerung der Auflösungszeit ergibt, wenn die Stärke als Sprengmittel in einer Zerfalls-Brausetablette eingesetzt werden soll. Es sind daher andere Substanzen vorzuziehen, wie:

### Beispiel 2:

Zu 500 Teilen Stärke wird eine Lösung von 130 Teilen Alkohol und 50 Teilen Adipinsäure im Vakuum bei 40° C eingesaugt, verteilt und anschliessend bei 40-50° C im Vakuum getrocknet.

Auch hierbei sinkt der ursprüngliche Wassergehalt der Stärke von 12% auf 7% ab. Diese beladene Stärke ist für ZerfallsBrausetabletten besonders gut geeignet, da die Adipinsäure schwerer wasserlöslich ist.

### Beispiele 3 und 4:

500 Teile Stärke werden mit einer Lösung von 100 Teilen Alkohol und 40 Teilen Weinsäure oder mit 150 Teilen Alkohol und 20 Teilen Fumarsäure wie unter Beispiel 1 beladen.

### Beispiel 5:

Man versetzt 500 Teile Stärke mit einer Lösung von 100 ml Alkohol und 5 g PVP K 90. Auch diese Stärke liefert gute Tablettenhärten und verlängert die Zerfalls- bzw. Lösezeit nicht.

## Patentansprüche (Patentansprüche für folgende(n) Vertragsstaat(en): AT, BE, DE, FR, GB, IT, NL, SE)

1. Stärke in Form von nicht hydrolysierten Starkekörnern, dadurch gekennzeichnet, dass sie mit 1 - 20 Gew.% einer essbaren organischen Säure aus der Gruppe Zitronensäure, Weinsäure, Adipinsäure, Fumarsäure oder mit einem längerkettigen, unverzweigten Polyvinylpyrrolidon beladen ist.

2. Stärke nach Anspruch 1, dadurch gekennzeichnet, dass sie mit 3 - 10 Gew.% der in Anspruch 1 definierten Substanz beladen ist.

3. Verfahren zur Herstellung der Stärke nach Anspruch 1 oder 2, dadurch gekennzeichnte, dass es folgende Verfahrensschritte beinhaltet:
a) Stärke wird in einem Vakuummischkessel auf 40-50°C erwärmt,
b) anschliessend wird die in einem Lösungsmittel gelöste, wie in Anspruch 1 definierte Substanz in den Vakuummischkessel eingesaugt und unter Mischen auf den Stärkekörnern gleichmässig verteilt,
c) danach wir die so beladene Stärke durch Wärme und/oder Vakuum getrocknet.

4. Verfahren nach Anspruch 3, dadurch gekennzeichnet, dass die Substanz unter schwingendem Mischen auf den Stärkekörnern verteilt wird.

5. Verfahren nach Anspruch 3 oder 4, dadurch gekennzeichnet, dass die Substanz in Alkohol gelöst ist.

6. Verwendung der Stärke nach Ansprüchen 1 und 2 als Hilfsstoff für pharmazeutische Zubereitungen in Tablettenform.

## Patentansprüche (Patentansprüche für folgende(n) Vertragsstaat(en): ES)

1. Verfahren zur Herstellung von Stärke, dadurch gekennzeichnet, dass Stärkekörner mit 1 - 20 Gew.% einer essbaren organischen Säure aus der Gruppe Zitronensäure, Weinsäure, Adipinsäure, Fumarsäure oder mit einem längerkettigen, unverzweigten Polyvinylpyrrolidon beladen werden.

2. Verfahren nach Anspruch 1, dadurch gekennzeichnet, dass die Stärkekörner mit 3 - 10 Gew.% der in Anspruch 1 definierten Substanz beladen werden.

3. Verfahren nach Anspruch 1 oder 2, dadurch gekennzeichnet, dass es folgende Verfahrensschritte beinhaltet:
a)Stärke wird in einem Vakuummischkessel auf 40-50°C erwärmt,
b)anschliessend wird die in einem Lösungsmittel gelöste, wie in Anspruch 1 definierte Substanz in den Vakuummischkessel eingesaugt und unter Mischen auf den Stärkekörnern gleichmässig verteilt,
c)danach wird die so beladene Stärke durch Wärme und/oder Vakuum getrocknet.

4. Verfahren nach Anspruch 3, dadurch gekennzeichnet, dass die Substanz unter schwingendem Mischen auf den Stärkekörnern verteilt wird.

5. Verfahren nach Anspruch 3 oder 4, dadurch gekennzeichnet, dass die Substanz in Alkohol gelöst ist.

6. Verwendung der Stärke nach Ansprüchen 1 und 2 als Hilfsstoff für pharmazeutische Zubereitungen in Tablettenform.

## Claims (Claims for the following Contracting State(s): AT, BE, DE, FR, GB, IT, NL, SE)

1. Starch in the form of anhydrolyzed starch grains, characterized in that it is laden with 1 - 20% by weight of an edible organic acid from the group consisting of citric acid, tartaric acid, adipic acid and fumaric acid or with a relatively long-chain, unbranched polyvinylpyrrolidone.

2. Starch according to Claim 1, characterized in that it is laden with 3 - 10% by weight of the substance defined in Claim 1.

3. Process for the preparation of the starch according to Claim 1 or 2, characterized in that it comprises the following process steps:
a) starch is heated to 40 - 50°C in a vacuum mixing vessel,
b) the substance as defined in Claim 1, dissolved in a solvent, is then sucked into the vacuum mixing vessel and is uniformly distributed over the starch grains with mixing and
c) the starch laden in this manner is then dried by means of heat and/or a vacuum.

4. Process according to Claim 3, characterized in that the substance is distributed over the starch grains with vibratory mixing.

5. Process according to Claim 3 or 4, characterized in that the substance is dissolved in alcohol.

6. The use of the starch according to Claims 1 and 2 as an excipient for pharmaceutical formulations in tablet form.

## Claims (Claims for the following Contracting State(s): ES)

1. Process for the preparation of starch, characterized in that starch grains are laden with 1 - 20% by weight of an edible organic acid from the group consisting of citric acid, tartaric acid, adipic acid and fumaric acid or with a relatively long-chain, unbranched polyvinylpyrrolidone.

2. Process according to Claim 1, characterized in that the starch grains are laden with 3 - 10% by weight of the substance defined in Claim 1.

3. Process according to Claim 1 or 2, characterized in that it comprises the following process steps:
a) starch is heated to 40 - 50°C in a vacuum mixing vessel,
b) the substance as defined in Claim 1, dissolved in a solvent, is then sucked into the vacuum mixing vessel and is uniformly distributed over the starch grains with mixing and
c) the starch laden in this manner is then dried by means of heat and/or a vacuum.

4. Process according to Claim 3, characterized in that the substance is distributed over the starch grains with vibratory mixing.

5. Process according to Claim 3 or 4, characterized in that the substance is dissolved in alcohol.

6. The use of the starch according to Claims 1 and 2 as an excipient for pharmaceutical formulations in tablet form.

## Revendications (Revendications pour l'(les) Etat(s) contractant(s) suivant(s): AT, BE, DE, FR, GB, IT, NL, SE)

1. Amidon, se présentant sous la forme de grains d'amidon non hydrolysés, caractérisé en ce qu'ils sont chargés, pour une teneur de 1 à 20 % en poids, d'un acide organique comestible issu du groupe de l'acide citrique, l'acide tartrique, l'acide adipique, l'acide fumarique ou d'une polyvinylpyrrolidone non ramifiée, à chaîne longue.

2. Amidon selon la revendication 1, caractérisé en ce qu'il est chargé, avec une quantité de 3 à 10 % en poids, de la substance définie à la revendication 1.

3. Procédé de préparation de l'amidon selon la revendication 1 ou 2, caractérisé en ce qu'il présente les étapes de procédé ci-après :
a) l'amidon est chauffé à une température de 40 à 50 °C, dans une chaudière de mélange travaillant sous vide,
b) la substance définie dans la revendication 1, dissoute dans un solvant, est ensuite introduite par aspiration dans la chaudière de mélange travaillant sous vide et répartie régulièrement sur les grains d'amidon, en procédant à un mélange,
c) l'amidon ainsi chargé est séché par action de chaleur et/ou de vide.

4. Procédé selon la revendication 3, caractérisé en ce que la substance est répartie sur les grains d'amidon, en effectuant un mélange faisant appel à des vibrations.

5. Procédé selon la revendication 3 ou 4, caractérisé en ce que la substance est dissoute dans de l'alcool.

6. Utilisation de l'amidon selon les revendications 1 et 2, à titre de produit auxiliaire pour des préparations pharmaceutiques et se présentant sous forme de comprimés.

## Revendications (Revendications pour l'(les) Etat(s) contractant(s) suivant(s): ES)

1. Procédé de fabrication d'amidon, caractérisé en ce que des grains d'amidon sont chargés, pour une teneur de 1 à 20 % en poids, d'un acide organique comestible issu du groupe de l'acide citrique, l'acide tartrique, l'acide adipique, l'acide fumarique ou d'une polyvinylpyrrolidone non ramifiée, à chaîne longue.

2. Procédé selon la revendication 1, caractérisé en ce que les grains d'amidon sont chargés avec une quantité de 3 à 10 % en poids, de la substance définie à la revendication 1.

3. Procédé selon la revendication 1 ou 2, caractérisé en ce qu'il présente les étapes de procédé ci-après :
a) l'amidon est chauffé à une température de 40 à 50 °C, dans une chaudière de mélange travaillant sous vide,
b) la substance définie dans la revendication 1, dissoute dans un solvant, est ensuite introduite par aspiration dans la chaudière de mélange travaillant sous vide et répartie régulièrement sur les grains d'amidon, en procédant à un mélange,
c) l'amidon ainsi chargé est séché par action de chaleur et/ou de vide.

4. Procédé selon la revendication 3, caractérisé en ce que la substance est répartie sur les grains d'amidon, en effectuant un mélange faisant appel à des vibrations.

5. Procédé selon la revendication 3 ou 4, caractérisé en ce que la substance est dissoute dans de l'alcool.

6. Utilisation de l'amidon selon les revendications 1 et 2, à titre de produit auxiliaire pour des préparations pharmaceutiques et se présentant sous forme de comprimés.
